(19) Europäisches Patentamt
European Patent Office
Office européen des brevets

(11) Veröffentlichungsnummer : **0 109 061**
**B1**

(12) # EUROPÄISCHE PATENTSCHRIFT

(45) Veröffentlichungstag der Patentschrift : **06.07.88**

(51) Int. Cl.[4] : **A 61 L 17/00**, A 61 L 27/00, A 61 C 13/09, A 61 K 6/02

(21) Anmeldenummer : **83111242.0**

(22) Anmeldetag : **10.11.83**

(54) **Implantate und Verfahren zu deren Herstellung.**

(30) Priorität : **10.11.82 DE 3241589**

(43) Veröffentlichungstag der Anmeldung : **23.05.84 Patentblatt 84/21**

(45) Bekanntmachung des Hinweises auf die Patenterteilung : **06.07.88 Patentblatt 88/27**

(84) Benannte Vertragsstaaten : **AT CH DE FR GB IT LI NL SE**

(56) Entgegenhaltungen :
**EP-A- 0 094 924**
**DE-A- 2 340 546**
**DE-A- 2 633 213**
**DE-A- 2 910 335**

(73) Patentinhaber : **Engelhardt, Achim, Dr.**
**Donnersbergstrasse 42**
**D-6000 Frankfurt 71 (DE)**

(72) Erfinder : **Engelhardt, Achim, Dr.**
**Donnersbergstrasse 42**
**D-6000 Frankfurt/M.-Niederrad (DE)**
Erfinder : **Zöphel, Gerd Peter, Dr.**
**Bruchfeldstrasse 16**
**D-6000 Frankfurt/M.-71 (DE)**

(74) Vertreter : **Kossobutzki, Walter, Dipl.-Ing. et al**
**Waldstrasse 6**
**D-5419 Helferskirchen (DE)**

**EP 0 109 061 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung bezieht sich auf ein Implantat als Ersatzteil für Skelette, Gelenke, Zähne, für Tumorprothesen oder ähnliche Teile des menschlichen oder tierischen Körpers, bestehend aus einem zumindest teilweise aus Metall gebildeten Kern, der zumindest teilweise mit einem ein- oder mehrschichtigen Überzug aus Keramik oder biologisch verträglichem und/oder bioaktivem Glas versehen ist, sowie auf ein Verfahren zu dessen Herstellung.

Es sind Implantate bekannt, die ganz oder teilweise aus Metall, insbesondere aus Edelstahl, aus Glaskeramik, Sinterkeramik, Osteoceram-Materialien oder besonderen Glasarten bestehen. Dabei haben Implantate aus Metall den Nachteil, daß sie nur einen ungenügenden Korrosions- und Spannungskorrosionswiderstand gegenüber den Säften und Sekreten des lebenden Körpers sowie einen unzureichenden Abriebwiderstand bei relativ aneinandergleitenden Implantatteilen besitzen. Der metallische Abrieb birgt die Gefahr in sich, daß derselbe in das umgebende Gewebe gelangen und sich dort einlagern kann, was zu ernsten Beeinträchtigungen, beispielsweise Reizerscheinungen und Gelenkversteifungen, und damit zu einer Verringerung der Lebensdauer des Implantates führt.

Um die vorerwähnten Nachteile zu verringern, sind aus der DE-OS 2 340 546 Implantate bekannt, die ganz oder teilweise mit Email überzogen sind. Dabei kann der Überzug aus teilweise kristallisiertem Email bestehen oder als mehrschichtiger Emailauftrag ausgebildet sein, wobei sich gegebenenfalls ein teilweise kristallisiertes Grundemail auf einem Basismetall befindet. Bei einem mehrschichtigen Auftrag des Emails ist es vorteilhaft, wenn ein glasiges Deckemail vorgesehen wird. In jedem Fall wird bevorzugt, daß die Emailoberfläche örtlich, beispielsweise durch mechanismes oder chemisches Aufrauhen oder durch Einschleifen von Vertiefungen, profiliert ist.

Auch bei derartigen bekannten Implantaten ist die Bindung zwischen dem Implantat und dem Gewebe, welches das Implantat nach dem Einsetzen in den menschlichen oder tierischen Körper umgibt, noch verbesserungsbedürftig. Dabei ist zu berücksichtigen, daß es wesentlich ist, daß die Verbindung zwischen dem Implantat und dem Gewebe Relativbewegungen im Grenzbereich ausschließen sollte. Zusätzlich müßte diese Verbindung so gestaltet sein, daß das durch eine Operation geschädigte oder unterbrochene biophysikalische und biochemische Regelsystem des Gewebes in seinen spezifischen Impulsmustern, die in aller Regel Stellreize für Wachstum und Erhaltung des Gewebes darstellen, durch stoffschlüssigen Verbund möglichst wieder hergestellt wird. Ferner ist durch Konstruktion und chemische Oberflächengestaltung zu berücksichtigen, daß auftretende mechanische Kräfte in optimaler Weise auf den Knochen verteilt und übertragen werden. Auch verschiedne bioelektrische und biochemische Eigenschaften, z. B. Piezoelektrizität und Oberflächengruppen des Knochenkollagens und Strömungspotentiale im Osteonensystem, die nach bisheriger Erkenntnis steuernd in den Knochenstoffwechsel eingreifen, sollen beim Implantat möglichst gut ersetzt bzw. erhalten werden.

Aus EP-A-94924, welches Dokument unter Artikel 54(3) EPÜ fällt, sind Implantate bekannt, die zumindest eine Oberfläche aus Glas, Silikon, Aluminium- oder Silikon-Kautschuk aufweisen, die ihrerseits mit einer bioverträglichen Schicht versehen ist. Die bioverträgliche Schicht besteht aus Dextranen mit mindestens einer Hydroxylgruppe und ist mittels Silanen mit der Oberfläche der Implantate verbunden. Dabei sind Dextrane Biopolymere auf der Basis von Kohlehydraten, die keine Proteine enthalten und somit keinerlei Wirkung auf die Knochenbildung haben. Durch die Beschichtung mit Dextranen soll eine Blutkoagulation bzw. Blutgerinnung vermieden werden ; eine chemische Verbindung wird daher nicht angestrebt, sondern verhindert. Das bedeutet, daß diese bekannten Implantate nur zur Bildung von Blutgefäßen verwendet werden können ; ihr Einsatz für Gelenk- und Skelettersatzteile ist somit ausgeschlossen.

Ausgehend von dem aus der DE-OS 2 340 546 bekannten Stand der Technik liegt der Erfindung die Aufgage zugrunde, ein Implantat als Ersatzteil für Skelette, Gelenke, Zähne, für Tumorprothesen oder ähnliche Teile des menschlichen oder tierischen Körpers zu schaffen, das nicht nur die Vorteile der bekannten Implantate, wie Verschleiß- und Korrosionsfestigkeit und große konstruktive Gestaltungsfreiheit bewahrt, sondern das darüber hinaus auch noch die Verbindung mit dem Verankerungsgewebe erheblich verbessert. Dabei sollen vor allem durch physiologisch angepaßte Krafteinleitung, insbesondere aber auch durch chemische Oberflächenmodifikation mikroskopische Relativbewegungen im Grenzbereich zwischen Implantat und Umgebungsgewebe weitgehend erhalten bleiben, so daß sich keine zur Kraftübertragung ungeeignete, präossäre unausgereifte Gewebeformationen ausbilden können.

Zur Lösung dieser Aufgabe wird bei dem Implantat der eingangs beschriebenen Gattung vorgeschlagen, daß auf dem Überzug eine mit demselben kovalent gebundene Schicht aus einer Organosiliziumverbindung vorgesehen ist, die eine weitere mit ihr mit oder ohne Kopplungsmolekül kovalent gebundene Schicht aus Strukturproteinen, Bruchteilen davon in Form von Polypeptiden oder Glykoproteinen trägt.

Die Erfindung umfaßt auch ein Verfahren zur Herstellung des oben angegebenen Implantats, bei dem ein zumindest teilweise aus Metall bestehender Kern, der zumindest teilweise mit einem ein- oder mehrschichtigen Überzug aus Keramik oder biologisch verträglichem und/oder bioaktivem Glas beschichtet wird, wobei der Überzug zuerst mit einer zusätzlichen Schicht aus einer Organosiliziumverbindung in kovalenter Bindung versehen wird, und anschließend auf diese Schicht eine weitere mit ihr kovalent gebundene Schicht aus Strukturproteinen oder Bruchteile davon in Form von Polypeptiden oder

Glykoproteinen gegebenfalls unter Verwendung eines geeigneten Kopplungsmittels, aufgebracht wird.

Das Substrat oder Basismaterial ist in seiner Formgebung und seinen mechanischen-physikalischen Kennwerten sehr genau und sicher auf die Erfordernisse des speziell vorliegenden Anwendungsfalls einstellbar. Sofern ein Implantat in verhältnismäßig kleinen Abmessungen und komplizierten Formen, beispielsweise mit scharfen Kanten oder Ecken, Rundungen mit kleinem Radius etc., hergestellt werden soll, empfiehlt es sich, ein Material mit einem geringen linearen Ausdehnungskoeffizient als Substrat zu verwenden.

Unter dem Begriff « Bioglas » werden in dieser Anmeldung Glasmaterialien verstanden, welche bekannterweise im biologischen Bereich, insbesondere für Implantate, verwendet werden können.

Es wurde bereits darauf hingewiesen, daß auch geeignete Materialien aus Keramik oder Bioglas für einen ein- oder mehrschichtigen Überzug des Substrats sowie zweckmäßige Verfahren zu deren Aufbringung bekannt sind. Es sei z. B. die bereits oben erwähnte Offenlegungsschrift 23 40 546 angeführt.

Allgemein kann gesagt werden, daß der Anteil an Siliziumverbindungen in dem Keramik- oder Glasmaterial in einem Bereich von mindestens 40 Gew.-%, insbesondere 50 bis 100 Gew.-% liegen soll.

So kommen als Glasmaterialien beispielsweise Quarzglas, Borsilikat-Gläser, Kalk-Natron-Gläser und als Glaskeramiken teil- oder vollkristallisierte Gläser bzw. Emails in Betracht. Als Sinterkeramik können beispielsweise Tonmaterialien (aus hauptsächlich Kaolin enthaltenden Massen hergestellt) oder Steinzeug verwendet werden.

Das mit einem Überzug aus Keramik oder Bioglas versehene Implantat wird nun an der Oberfläche aktiviert, d. h. daß eine optimale Anzahl von —Si—OH-Gruppen an der Oberfläche vor der Behandlung mit der Organosiliziumverbindung geschaffen wird.

Insbesondere wenn polierte Glas- oder Keramikoberflächen des überzogenen Substrats vorliegen, ist es zweckmäßig, diese mit Fluorwasserstoff vorzuätzen, und anschließend mit reinem Wasser zu hydrolysieren. Diese Behandlung kann dadurch vorgenommen werden, daß man 10-%igen Fluorwasserstoff bei Raumtemperatur einwirken läßt.

Unabhängig von der Beschaffenheit der Glas- oder Keramikoberfläche ist folgende Arbeitsweise für die Aktivierung von dieser zweckmäßig :

Alle Oberflächen werden in einem staubfreien, sauerstofffreien Raum mit folgenden ultrafiltrierten, entgasten Lösungsmitteln gereinigt : Aceton, Toluol, Äthanol und zweifach destilliertes Wasser. Danach wird die fett- und staubfreie Oberfläche mit einem Dampfstrom aus einem organischen Lösungsmittel, das mit Wasser ein azeotropisches Gemisch bildet, in bekannter Weise getrocknet. Danach erfolgt eine oxidative Hydrolyse der Oberfläche mit 1-3-m Salpetersäure, die man 3 Stunden bei Raumtemperatur einwirken läßt. Es schließt sich eine Waschstufe mit verdünnter Salzsäure und dann mit reinem Wasser an. Danach kann sich eine saure Hydrolyse im Wasserbad bei erhöhten Temperaturen (beispielsweise bei 70 °C und etwa 5 bis 10 Stunden anschließen). Dafür wird zweckmäßig ortho-Phosphorsäure, Essigsäure, Perchlorsäure oder Salzsäure, zweckmäßig in einer Stärke von 1-3 m, vorgenommen, wonach eine kurze Wässerung mit Wasser erfolgt.

Danach wird eine weitere alkalische Hydrolyse mit erhitzter Natronlauge oder Kalilauge durchgeführt. Bei einer Alkalikonzentration von 0,5-3 M können beispielsweise 70 °C angewendet und die Hydrolyse über 24 Stunden durchgeführt werden.

Danach erfolgt zweckmäßig die Behandlung mit ultrafiltriertem, zweifach destilliertem Wasser.

Ziel der beschriebenen Behandlung der Oberfläche des Glas- oder Keramiküberzugs ist es, eine möglichst weitgehende, vorzugsweise quantitative, hydrolytische Spaltung von Oberflächenoxiden zu erreichen. Dabei entstehen vorwiegend Silanolgruppen ≡ Si—OH bzw. ≡ Si—O—Alkali.

Selbstverständlich können sämtliche für diesen Zweck geeignete bekannten Verfahren, insbesondere die Hydrolyse mit Säuren und/oder Alkalien angewendet werden. Derartige Verfahren können je nach dem vorliegenden speziellen Fall, z. B. der Art des Glas- oder Keramikmaterials, vom Fachmann ohne erfinderisches Zutun ausgewählt werden.

Die aktivierte Glas- bzw. Keramikoberfläche wird nun mit einer Organosiliziumverbindung, z. B. auf der Basis eines Di- oder Trialkoxysilans, behandelt, wobei der organische Rest kovalent an die Silanolgruppen der Oberfläche angekoppelt wird. Vorzugsweise enthält diese Organosiliziumverbindung Alkylamino- und/oder Alkylhalogenidgruppe(n), und/oder Glycid-oxy- bzw. Glycerylgruppe(n), insbesondere solche mit 1 bis 4 Kohlenstoffatomen. Die Art der entstehenden Verbindungen ist nicht erfindungswesentlich, jedoch wird bevorzugt, daß eine möglichst feste Verbindung zwischen der Oberfläche der silikatischen Werkstoffe und der Organosiliziumverbindung entsteht.

Die entstehenden Oberflächengruppen sind durch übliche Verfahren der präparativen organischen Chemie, insbesondere Reduktion oder Oxidation in eine aktivierte Form überführbar und können mit körperverträglichen organischen Polymeren oder auch Fasern gekoppelt werden.

Entsprechend einer bevorzugten Ausführungsform der vorliegenden Erfindung werden als Organosiliziumverbindungen (Silane) Dialkoxyalkyl- oder Trialkoxysilane, insbesondere der allgemeinen Formel

$$(R^1O)_3\ Si—R^2 \text{ oder } (R^1O)_2\ SiR^2R^3$$

verwendet,

worin $R^1$ und $R^3$ unabhängig voneinander eine Alkylgruppe, insbesondere mit 1-4 Kohlenstoffatomen, z. B. mit 1-2 Kohlenstoffatomen, und $R^2$ eine gerad- oder verzweigtkettige Aminoalkyl-, Alkylhalogenid-, Thiol- oder einen mit mehreren Hydroxylgruppen substituierten Alkylrest bzw. die Glycid-oxygruppe, insbesondere mit 2-5 Kohlenstoffatomen bedeuten.

Zur Silanisierung können mit gutem Erfolg folgende Organosiliziumverbindungen verwendet werden :

$(CH_3O)_3$-Si-$(CH_2)_{2,3}$ od. $_4$-$NH_2$

$(CH_3O)_3$-Si-$(CH_2)_{2,3}$ od. $_4$-Cl

$(CH_3O)_3$-Si-$(CH_2)_{2,3}$ od. $_4$-SH

$(CH_3O)_3$-Si-$(CH_2)_{2,3}$ od. $_4$-OH

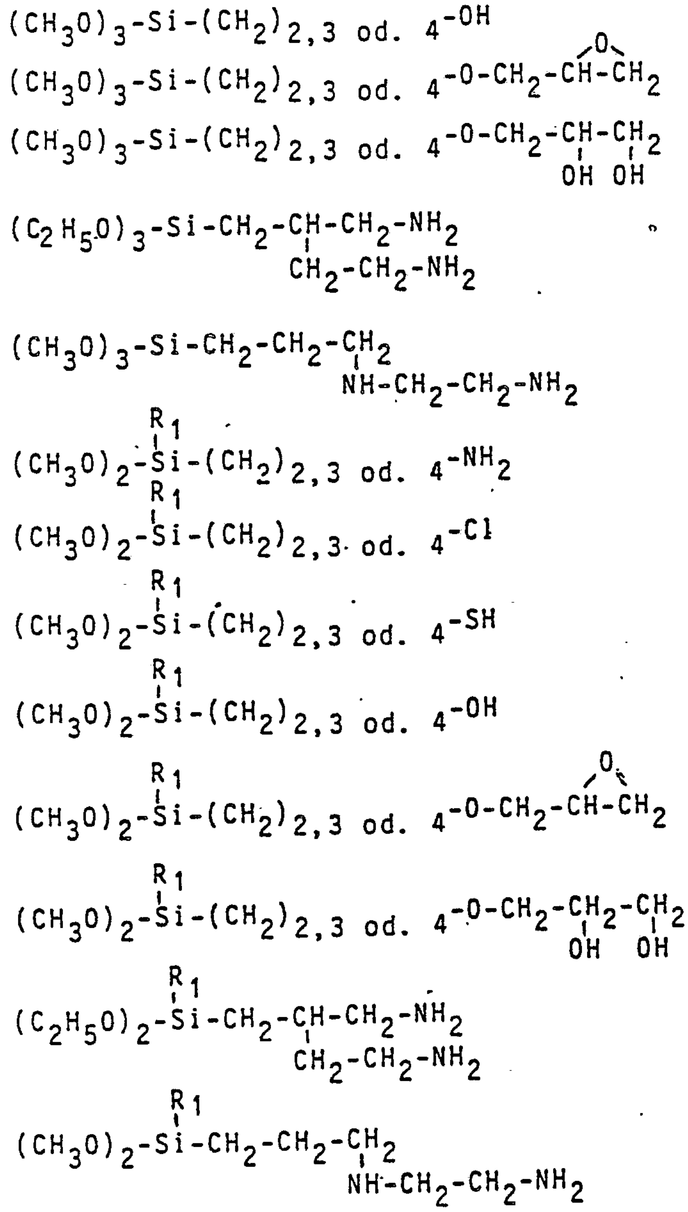

In den obigen Formeln hat $R_1$ die auf Seite 7 angegebene Bedeutung.

In den oben genannten Organosiliziumverbindungen können selbstverständlich die angegebenen Methoxy- bzw. Äthoxygruppen durch eine andere Alkoxygruppe, d..h. also durch eine Methoxy-, Äthoxy-, Propoxy- bzw. Butoxygruppe, ersetzt werden.

Die Silanisierung erfolgt zweckmäßig unter einer staubfreien Atmosphäre, wobei eines der drei folgenden Verfahren, je nach gewünschter Schichtdicke und Schichtqualität zur Anwendung kommt :

1. Gasphasenbeschichtung (ergibt homogene, dünnere Schichten)

Die verunreinigten aktivierten Oberflächen aus Glas- oder Keramikmaterialien werden in einem

Reaktor aus Duranglas, gegebenenfalls unter einer inerten Atmosphäre mit Dampf einer Mischung von 1 bis 2 Volumenteilen der Organosiliziumverbindung mit 13 bis 15 Volumenteilen eines aprotischen, mäßig polaren Lösungsmittels unter Erwärmung behandelt. Die Wärmezufuhr wird so geregelt, daß die zu behandelnden Oberflächen etwa 16 bis 20 Stunden vollkommen dem Dampf aus Silan und Lösungsmittel ausgesetzt werden. Als Lösungsmittel kann beispielsweise Toluol, Xylol, Tetrahydrofuran, Chloroform oder Dioxan verwendet werden.

Danach werden die silanierten Oberflächen vorzugsweise mehrfach gewaschen und getrocknet.

2. Beschichtung in organischer Lösung (ergibt etwas inhomogenere, dickere Schichten)

Die aktivierten Oberflächen werden, wie oben, einem Gemisch von 5-15 % des Silans im o. a. Lösungsmittel unter Zugaben von stöchiometrischen Mengen Wasser über 2 Stunden für 16-20 Stunden unter Rühren silanisiert.

3. Beschichtung in wässriger Lösung (ergibt stabile, dicke Polymerenschichten)

Das Silan wird vor der Beschichtungsreaktion langsam unter Rühren, je nach Ausgangs-pH nach üblichen chemischen Verfahren mit 0.1 M NaOH oder 0.1 M HCl so in frisch destilliertes Wasser eingetropft, daß der pH zwischen 5.5 und 6.0 bleibt und eine 5-10 % wässrige Lösung des Silans entsteht.

Danach wird die Mischung rasch auf 90 °C erhitzt, und das zu beschichtende Material wird 30 Min. unter leichtem Rühren in der Lösung belassen, danach mit frischem Wasser, Alkohol und Aceton gewaschen und bei 110 °C getrocknet.

Diese Waschbehandlung ist auf allen silanisierten Flächen anwendbar.

Erfindungsgemäß ist nun gefunden worden, daß die so erhaltenen endständigen Amino-, Sulfhydryl-, Halogen-, Glycidoxy- bzw. Hydroxylgruppen der siliziumorganischen Verbindung auf der behandelten Glas- oder Keramikschicht des Implantats zur Kopplung (direkt oder nach chemischer Modifikation der Gruppen durch Reduktion, Oxydation oder Substitution) mit Strukturproteinen oder ähnlichen Polypeptiden über eine Amid-, Peptid-, Imino-, Disulfid-, Äther- (oder andersgeartete kovalente) Bindung geeignet ist.

Zweckmäßig werden als Peptidmaterialien Grundbausteine, Teilsequenzen oder Mischfraktionen insbes. von natürlichen Peptiden aus der Klasse der Kollagene vom Typ I (Haut-, Sehne-, Knochen-Kollagene) herangezogen. Als Aminosäuresequenzen können beispielsweise Aminosäure-Triplets verwendet werden, die z. B. Glycin-, Prolin-, Hydroxyprolin- und/oder Alanin-Reste enthalten, wobei vorzugsweise der Glycinrest endständig ist. Weiterhin sind mit gutem Erfolg Kombinationen aus diesen Triplets möglich, wie z. B. entsprechende Hexa- und Nonapeptide.

Bei einer anderen Ausführungsform der Erfindung wird als Polypeptid ein solches mit einer $\alpha_1$-Kette oder deren Brom-Cyan-Spaltprodukte (CNBr-Peptide) eingesetzt.

Zum Aufbau von Polypeptidketten unter Ausbildung von Peptid-Bindungen zwischen einer C-terminal geschützten, N-terminal freien Aminosäure und einer Boc-geschützten, C-terminal freien Aminosäure oder eines Peptids eignet sich folgendes Verfahren:

0,1 M des C-terminalen freien Peptids werden in 400 ml eines geeigneten Lösungsmittels, wie Dichlormethan oder Dioxan oder THF, suspendiert und auf 0 °C abgekühlt. Es werden nacheinander zugefügt:

13,51 g (0,1 M) N-Hydroxybenzotriazol (HOBt)

30,94 g (0,1 mM) Dicyclohexylcarbodiimid (DCC) oder Diisopropylcarbodiimid.

Anschließend werden (0,1 M) der N-terminalen freien Aminosäure oder einer entsprechenden Menge des oberflächenbelegten Materials hinzugefügt und eine Stunde bei 0 °C und drei Stunden bei Raumtemperatur gerührt. Danach wird das Dichlormethan oder das entsprechende Lösungsmittel vollständig einrotiert und der Rückstand in 500 ml Essigester aufgenommen. Zur Abtrennung des bei der Reaktion anfallenden Harnstoffes wird wiederholt mit Lösungsmittel gespült und das Lösungsmittel dabei filtriert.

Die beschichtete Oberfläche wird mehrfach mit 5 %-iger Zitronensäure-Lösung, gesättigter Kochsalz-, 5 %-iger Natriumbicarbonat- und nochmals gesättigter Kochsalzlösung gewaschen und über Natriumsulfat getrocknet. Zur weiteren Abtrennung des Dicyclohexyl- oder Diisopropyl-Harnstoffes wird die Oberfläche mit Essigester gespült.

In einer anderen, prinzipiell ähnlichen Form der Peptidkopplung wird die trägergebundene Carboxylgruppe mit einem C-terminal geschützten Peptid nach dem oben angegebenen Verfahren gekoppelt.

In einem dritten Verfahren wird beispielsweise die freie Aminogruppe des auf der Glaskeramik mit ca. 40 $cm^2$ Oberfläche kovalent gebundenen Silans durch 18-stündiges Rühren bei Raumtemperatur in 100 ml (0,25 %) frisch gereinigte Glutaraldehydlösung zur Schiff'schen Base mit freier Aldehydfunktion umgesetzt. Nach dem Waschen mit 1 bis 2 Liter frischem Wasser wird durch Eintauchen der kurz im Vakuumexsicator getrockneten Oberfläche in eine 0,8 %-ige Lösung von Kollagen Typ 1 (Rindersehnen) in Phosphatpuffer (pH 8.0) mit 0,165 Mol NaCl und 0,024 Mol $K_2HPO_4$ eine Fällung von rekonsituierten Kollagenfasern auf der Oberfläche erzeugt. Nach Waschen in reichlich Wasser wird die Oberfläche mit 6 mMol $NaBH_3CN$ in 100 ml wässriger Lösung reduziert zur Stabilisierung der neugebildeten Schiff'schen Basen aus Oberflächengruppe und Peptid.

Nach Bedarf kann die Kollagenschicht durch 6 bis 8-stündiges Eintauchen in 0,25 % wässrige

Glutaraldehydlösung fixiert werden. Es schließt sich eine sorgfältige Waschung mit destilliertem $H_2O$, Aceton, DMSO und Vakuumtrocknung an. Die so beschichteten Oberflächen können durch Heißluft 180 °C, 30 Min. sterilisiert werden.

In einem weiteren Verfahren wird die durch Silanisierung auf der Oberfläche erzeugte Glycidoxypropyl-Gruppe durch nukleophilen Angriff einer —SH, $NH_2$ oder —OH Gruppe, beispielsweise aus einer Seitenkette eines Peptids, bei einem pH-Wert zwischen 8 und 10 (Phosphatpuffer) umgesetzt.

In einer anderen Ausführungsform des -selben Verfahrens wird die Glycidoxygruppe auf ca. 50 $cm^2$ Oberfläche durch einstündiges Rühren bei Raumtemperatur mit 100 ml $10^{-3}$ M HCl zur Glycerylgruppe hydrolysiert. Die gleiche Oberfläche wird mit Wasser gewaschen und mit 50 ml 0,1 M $NaIO_4$ in Wasser zwei Stunden bei Raumtemperatur geschüttelt. Nach dem Waschen mit Wasser wird die entstandene Aldehydfunktion, z. B. mit dem Aminorest aus der Seitenkette eines Peptids, reduktiv gekoppelt.

Dazu wird ca. 50 $cm^2$ Oberfläche mit 20 mg Peptid in 100 ml 0,2 M Phosphatpuffer von pH 6,0 mit 1 mM $NaBH_3CN$ 4 Tage bei Raumtemperatur langsam gerührt. Nach dem Waschen können nicht umgesetzte Aldehydfunktionen durch Reduktion mit 1 ml 0,5 M $NaBH_4$ bei 4 °C (15 Stunden) beseitigt werden.

Durch die genannten Kopplungsverfahren gelingt es, ausgehend von verschiedenen Grundfunktionen auf der Glasmatrix, Peptide bzw. Strukturеiweiße oder Bruchstücke davon, bzw. auch Glykoproteine (Proteoglykane) auf der Oberfläche kovalent zu fixieren.

Die so erhaltenen Implantate können in gleicher oder in ähnlicher Weiser wie entsprechend bekannte Implantate eingesetzt werden.

## Patentansprüche

1. Implantat als Ersatzteil für Skelette, Gelenke, Zähne, für Tumorprothesen oder ähnliche Teile des menschlichen oder tierischen Körpers, bestehend aus einem zumindest teilweise aus Metall gebildeten Kern, der zumindest teilweise mit einem ein- oder mehrschichtigen Überzug aus Keramik oder biologisch verträglichem und/oder bioaktivem Glas versehen ist, dadurch gekennzeichnet, daß auf dem Überzug eine mit demselben kovalent gebundene Schicht aus einer Organosiliziumverbindung vorgesehen ist, die eine weitere mit ihr mit oder ohne Kopplungsmolekül kovalent gebundene Schicht aus Strukturproteinen, Bruchteilen davon in Form von Polypeptiden oder Glykoproteinen trägt.

2. Implantat nach Anspruch 1, dadurch gekennzeichnet, daß die zusätzlichen Schichten zumindest an den Stellen vorgesehen sind, an denen zum Zweck einer guten stoffschlüssigen Verbindung Kontakt mit biologischem Gewebe, insbesondere Kollagen angestrebt wird.

3. Verfahren zur Herstellung eines Implantats nach Anspruch 1 oder 2, bei dem ein zumindest teilweise aus Metall bestehender Kern zumindest teilweise mit einem ein- oder mehrschichtigen Überzug aus Keramik oder biologisch verträglichem und/oder bioaktivem Glas beschichtet wird, dadurch gekennzeichnet, daß der Überzug zuerst mit einer zusätzlichen Schicht aus einer Organosiliziumverbindung in kovalenter Bindung versehen wird, und daß anschließend auf diese Schicht eine weitere mit Ihr kovalent gebundene Schicht aus Strukturproteinen oder Bruchteilen davon in Form von Polypeptiden oder Glykoproteinen gegebenenfalls unter Verwendung eines geeigneten Kopplungsmittels, aufgebracht wird.

## Claims

1. Implant as a replacement part for skeletons, joints, teeth, for tumour protheses or similar parts of the human or animal body consisting of a core made, at least in part, of metal which is covered at least in part with a single- or several-layered coating made of ceramics or biologically compatible and/or bioactive glass, characterised by the fact that the coating has the same covalently bound layer made of an organosilicon compound which carries a further layer covalently bound with it with or without coupling molecules made out of structural proteins, fractions of them in the form of polypeptides or glycoproteins.

2. Implant in accordance with claim 1, characterised by the fact that the additional layers are provided at the points on which contact with biological tissue, in particular collagens, is aimed at for the purpose of a well sealed bond between the materials.

3. Procedure for the production of an implant in accordance with claim 1 or 2 in which case a core consisting of metal at least in part is covered with a single- or several-layered coating, at least in part, made of ceramics or biologically compatible and/or bioactive glass, characterised by the fact that the coating is covalently bound with an additional layer made out of an organosilicon compound and that a further covalently bound layer made of structure proteins or fractions of them in the form of polypeptides or glycoproteins (if necessary with the application of a suitable coupling agent) is attached.

## Revendications

1. Implant pour remplacer des pièces de squelettes, articulations, dents, comme prothèse de tumeur

ou des parties analogues du corps humain ou animal, comprenant une âme faite du moins en partie en métal, recouverte du moins en partie d'un revêtement à une ou plusieurs couches en céramique ou en verre de tolérance biologique et/ou de verre bioactif, caractérisé par le fait qu'il est prévu sur le revêtement une couche de liaison d'organosilicium, covalente avec le revêtement, portant elle-même une couche supplémentaire covalente de protéines structurales, dont partie d'entre elles sous forme de polypeptides ou de glycoprotéines, liée avec le revêtement avec ou sans molécule d'accouplement.

2. Implant selon revendication 1, caractérisé par le fait, que les couches supplémentaires sont du moins prévues là où l'on désire favoriser le contact avec les tissus biologiques, en particulier avec le collagène, pour permettre une bonne liaison matérielle.

3. Procédé de fabrication d'un implant selon revendication 1 ou 2, suivant lequel une âme constituée du moins partiellement en métal est recouverte d'un revêtement à une ou plusieurs couches de céramique ou d'un verre de tolérance biologique ou d'un verre bioactif, caractérisé par le fait que le revêtement est d'abord recouvert d'une couche supplémentaire qui consiste en une liaison d'organosilicium covalente, et que cette couche est recouverte d'une autre couche liée de façon covalente, de protéines structurales ou dont partie de celles-ci sous forme de polypeptides ou glycoprotéines, éventuellement en se servant d'un moyen d'accouplement adéquat.